# EUROPEAN PATENT SPECIFICATION

(11) **EP 3 169 286 B1**
(45) Date of publication and mention of the grant of the patent: **28.02.2018**
(21) Application number: 15738106.2
(22) Date of filing: 16.07.2015
(51) Int. Cl.: A61F 5/445

(54) **OSTOMY BAG**
OSTOMIEBEUTEL
POCHE DE STOMIE

(30) Priority: 17.07.2014 GB 201412693
(43) Date of publication of application: 24.05.2017
(73) Proprietor: WELLAND MEDICAL LIMITED, Crawley West Sussex RH10 9AS (GB)
(72) Inventor: SMITH, Rory James Maxwell, Crawley West Sussex RH10 9AS (GB); BRAY, Christopher David, Crawley West Sussex RH10 9AS (GB); FRAMPTON, Kim Sarah, Crawley West Sussex RH10 9AS (GB); NEWTON, Mark Andrew, Crawley West Sussex RH10 9AS (GB)
(74) Representative: Hutchins, Michael Richard
(86) International application number: PCT/EP2015/066278
(87) International publication number: WO 2016/008981

(56) References cited:
- GB-A- 2 290 713
- US-A- 5 690 622
- US-B2- 8 211 072

## Description

This invention relates to drainage bag assemblies, such as ostomy bags, for receiving bodily waste, and more particularly to an ostomy bag assembly comprising outer and inner bags that can be detached from an adhesive flange for disposal.

### Background of the Invention

Ostomy bags for receiving bodily waste from colostomy and ileostomy patients are well known. One of the problems faced by users of ostomy bags, particularly colostomy bags, is how to dispose of the contents of the bag.

Many known forms of ostomy bag are made from materials that are not biodegradable and are not easily flushed down a W.C. because of, for example, the buoyancy and relative bulk of the bags. With non-flushable bags, it has been common practice to cut an edge of the bag and then deposit the contents of the bag in the W.C. for flushing away, leaving the soiled bag for separate disposal, e.g. by incineration or by wrapping and placing in a waste bin.

One solution to this problem has been to provide ostomy bags made from materials that are capable of being flushed down a W.C. and examples of such bags are disclosed in WO 94/12128, EP 0259184, US 2004/0059306, EP 0320895, US 5,989,235, GB 2083762, EP 388924, GB 2227668, GB 2193925 and WO 2007/085803.

In many cases, the flushable ostomy bag comprises an inner bag which is formed from a material that disintegrates or dissolves in water or is otherwise disposable and a protective outer bag formed from a material that is resistant to water. The outer bag can be constructed so as to be reusable several times, means being provided for opening the outer bag to permit removal and replacement of the inner bag or liner. The outer and inner bags may both be attached, directly or indirectly, to an adhesive flange which comprises a layer of a bio-compatible adhesive such as a hydrocolloid adhesive to secure the ostomy bag to the body of the patient about the stomal opening.

US 2004/0059306 in particular describes several forms of construction of two piece ostomy bags in which the inner bag or liner is replaceable and a re-fastenable opening is provided in the outer bag to give access to the inner bag so that it can be replaced.

US 5,785,695 (Alcare) discloses ostomy appliances comprising inner and outer bags that are releasably attached to an adhesive flange by means of mechanical couplings comprising coupling rings having annular grooves that engage corresponding annular rims on the adhesive flange to form snap-fit connections.

US 2003/0153883 (Hansen) discloses ostomy appliances comprising an adhesive flange to which is secured a first mechanical coupling ring for the attachment of an outer bag. An inner bag or liner can also be secured to the first mechanical coupling ring by means of a second mechanical coupling ring which encircles the mouth of the inner bag and which forms a snap-fit connection against the radially inner surface of the first mechanical coupling ring.

A problem with ostomy appliances employing coupling rings to connect the inner and outer bags to an adhesive flange is that the coupling rings almost invariably make the appliance stiffer and less flexible and hence less comfortable to wear. In addition, where the coupling rings for the inner and outer bags are placed relatively close together, this can make separation and replacement of the bags difficult, particularly for people with impaired or reduced manual dexterity. A further problem with using coupling rings is that they will need to be removed prior to disposal of an inner bag down a WC. Not only does this add an additional potentially awkward step to the removal and disposal process but it may also result in the user's hands coming into contact with faecal waste at the mouth of the bag.

As an alternative to using mechanical couplings, adhesive bonding has been used to secure the inner and outer bags to the adhesive flange. Examples of ostomy bags making use of adhesive bonding can be found in US 5,865,819 (Hollister) and WO 2004/082452 (Coloplast).

US 5,865,819 discloses an arrangement in which the inner and outer bags each have their own separate adhesive flange for direct connection to the body of the patient.

WO 2004/082452 discloses ostomy bags comprising an adhesive flange for attachment to the body of a patient, and inner and outer bags. The inner and outer bags are each provided with adhesive rings for attachment to the adhesive flange. In the preferred ostomy bag constructions disclosed in WO 2004/082452, the outer diameter of the adhesive ring of the inner bag is larger than the inner diameter of the adhesive ring of the outer bag and hence there is overlap between the two adhesive rings.

US 5,690,622 (Smith et al) and WO 2007/085803 (Welland Medical Limited) disclose ostomy bag assemblies comprising inner and outer bags secured to an adhesive flange.

GB 2290713 (Welland Medical Limited) discloses an ostomy bag liner comprising biodegradable inner and outer walls which are arranged in an unconnected non-laminar construction, but sealed together at the peripheral margins and around a flange.

EP2289471 and US 8211072 (Welland Medical Limited) - which is seen as the closest prior art document - an ostomy bag assembly comprising outer and inner bags secured to one side of an adhesive flange wherein the outer bag is detachably bonded to an attachment zone on the polymeric backing film of the flange by means of an annular bonding element which is interposed between the outer bag and the attachment zone.

### The Invention

The present invention provides an improved ostomy bag assembly which benefits from ease of manufacture and provides more options for disposal than many known bags.

More particularly, the invention provides an ostomy bag having an adhesive flange to which are attached an inner bag for receiving stomal waste and a protective outer bag wherein both the inner and outer bags can be peeled away from the adhesive flange to allow for separate disposal of the flange and bags. Accordingly, in a first aspect, the invention provides an ostomy bag assembly comprising outer and inner bags secured to one side of an adhesive flange; the adhesive flange having means defining an orifice to enable bodily waste from a stomal opening to be received by the inner bag; wherein the adhesive flange is of laminar construction and comprises, in sequence, from a body-contacting surface outwards:
(a) a bioadhesive layer for securing the flange to a body surface of a patient about the stomal opening;
(b) a first polymeric support layer attached to the bioadhesive layer (a);
(c) a heat sealable layer having a heat sealable adhesive surface which releasably bonds the heat sealable layer to the first polymeric support surface;
(d) a second polymeric support layer bonded to the heat sealable layer;
(e) a weldable polymeric layer to which the inner and outer bags are attached, the weldable polymeric layer being bonded to the second polymeric support layer;
the adhesive flange having an annular channel which surrounds the means defining the orifice and extends through layers (c), (d) and (e) so as to form radially inner and outer attachment zones separated by the annular channel, the inner bag being attached to the radially inner attachment zone and the outer bag being attached to the radially outer attachment zone;
and wherein the inner and outer bags, together with attached portions of layers (c), (d) and (e) can each be peeled away from the first polymeric support layer (b).

In use, the ostomy bag assembly of the invention is attached to the body surface of a patient about a stoma so that bodily waste from the stoma can be collected by the inner bag. When the inner bag is full, or it is otherwise desired to replace the ostomy bag assembly with a new one, the assembly may be removed for disposal. The outer bag, which typically is still relatively clean, together with the attached portions of layers (c), (d) and (e) can be peeled away from the first polymeric support layer (b) and disposed of in household or other waste. The inner bag, containing bodily waste, together with the attached portions of layers (c), (d) and (e), can then also be peeled away from the first polymeric support layer and disposed of by flushing down a WC. The remaining part of the adhesive flange can be disposed of in normal household waste. Alternatively, the inner bag can be left on the flange and disposed of by flushing down a WC. Thus one advantage of the ostomy bag assembly of the invention is that it provides the patient with greater flexibility when disposing of the used assembly. For example, if the assembly is heavily soiled, the patient can peel away only the outer bag and then flush the remainder of the flange with inner bag attached down the WC. On the other hand, if the assembly, or at least the adhesive flange part of the assembly, is only lightly soiled, both inner and outer bags can be peeled away from the flange and only the inner bag and its waste contents flushed down the WC.

The ostomy bag assemblies of the invention typically are provided with a protective covering layer (f), e.g. a siliconised release paper, which protects the bioadhesive layer (a) and which is removed prior to fitting the ostomy bag assembly to the patient.

The bioadhesive layer (a) is a layer of an adhesive which forms a good bond to the skin, is skin-friendly and most preferably causes negligible adverse skin reactions under normal conditions of use. The bioadhesives used for layer (a) can be, for example, any of the bioadhesives commonly used for fitting ostomy bags to patients. Particular bioadhesives that can be used in the ostomy bag assemblies of the present invention are hydrocolloid adhesives. Such adhesives are well known and widely used and a detailed description of their properties is not required here. However, one form of hydrocolloid adhesive that may be used in the ostomy bag assemblies of the present invention comprises powdered gelatin, pectin and cellulose in a polyisobutylene (PIB) matrix. The powdered gelatin, pectin and cellulose absorb moisture and gel within the matrix. An adhesive flange of this type can be formed by mixing together PIB and powder components for a period of about 45 minutes at 60 to 75 °C and then extruding the mixture for further processing.

The adhesive flange has means defining an orifice to enable bodily waste from a stomal opening to be received by the inner bag. The means defining the orifice can be a hole, or one or more lines (e.g. concentric lines) indicating where a hole should be made, or a combination of a hole and one or more lines (e.g. circular lines concentric with the hole) indicating enlarged hole diameters. Thus, a patient or medical professional can select the desired size of the hole on the adhesive flange depending on the characteristics of the stoma.

The heat sealable layer (c) acts as a peelable but non-repositionable adhesive. The term "non-repositionable" as used herein means that once the bag has been peeled away from the attachment zone, it is not possible to reattach it to the attachment zone by finger pressure alone. Thus, the adhesive is one which does not retain any adhesive capability at ambient temperature after the two surfaces to which it is bonded have been peeled apart.

The peelable non-repositionable adhesive can be a hot-melt adhesive. The hot-melt adhesive can be a thermoplastic polymer that has a lower melting point than the polymer from which the first polymeric support layer is formed. Typically, the difference in melting points between the first polymeric support layer and the hot-melt adhesive will be of the order of at least 20 °C.

Examples of materials functioning as hot-melt adhesives include ethylene vinyl acetate (EVA) and polyethylene, with EVA being preferred. The heat sealable layer may comprise an EVA layer having a coating of an ethylene vinyl acetate copolymer adhesive thereon which provides the heat sealable adhesive surface. Alternatively, the heat sealable layer can comprise a layer of ethylene vinyl acetate copolymer adhesive (e.g. in the form of an emulsion) which is in direct contact with the second polymeric support layer.

It will be appreciated that the strength of the bond between the heat sealable layer (c) and the first polymeric support layer (b) is typically less than the strengths of the bonds between the inner and outer bags to the weldable polymeric layer (e) or the strengths of the bonds between the layers (c), (d) and (e).

Thus, when the inner and outer bags are peeled away from the adhesive flange, it is the bond between the heat sealable layer (c) and the first polymeric support layer (b) that is broken. The inner and outer bags are each removed from the flange along with their attached portions of layers (c), (d) and (e).

The first polymeric support layer comprises a layer of polyurethane or polyamide film. Preferably, the polymeric backing film comprises a layer of polyurethane, and more preferably consists of a single layer of polyurethane film.

The second polymeric support layer is selected from polymers that are compatible with the polymers from which the weldable polymeric layer (e) and the heat sealable layer (c) are formed; i.e. are capable of forming a strong bond to the polymers of layers (e) and (c) during coextrusion to form a laminate. The second polymeric support layer (d) is typically of greater tensile strength than the polymers from which the layers (e) and (c) are formed.

The second polymeric support layer can be formed from, for example, a polyamide or an ethylene/methacrylic acid co-polymer or an ionomeric form thereof.

In one embodiment, the second polymeric support layer is formed from a polyamide.

A particular example of a material suitable for use as the second polymeric support layer is Surlyn®.

The weldable polymeric layer (e) is formed from a polymer that is capable of being welded to the outer bag, or a component part thereof, and optionally the inner bag or a component part thereof. The weldable polymeric layer (e) may be formed from, for example, an ethylene polymer or copolymer such as ethylene vinyl acetate copolymer.

The combined thicknesses of the layers (c), (d) and (e) can be, for example, from 120 µm to 180 µm, more typically from 140 µm to 160 µm, for example approximately 150 µm.

A particular example of the multilayer polymeric material is the PerfecSeal® coated PerfecFlex® medical forming film available from Perfecseal Limited of Londonderry, Northern Ireland, UK.

The outer bag may comprise outer and inner pairs of panels welded together around their peripheries, the inner pair of panels serving to provide a waterproof and odour-proof containment for the inner bag and the outer pair of panels serving as a comfort layer. The comfort layer may typically be formed from a non-woven fibrous material such as a non-woven polyethylene fabric formed from polyethylene fibres.

The outer bag can be formed from a multilayer polymeric film comprising an outer layer formed from a polymer which is weldable to the weldable polymeric layer (e) of the adhesive flange. For example, when the weldable polymeric layer (e) is formed from an ethylene polymer or copolymer such as ethylene vinyl acetate, the outer layer of the multilayer polymeric film may also be formed from an ethylene polymer or copolymer such as ethylene vinyl acetate. In one particular embodiment, both the weldable polymeric layer (e) and the outer layer of the multilayer polymeric film of the outer bag can be formed from ethylene vinyl acetate copolymer.

In one embodiment, the outer bag comprise an outer layer of ethylene vinyl acetate and a layer of polyvinyl dichloride or polyvinyl chloride.

For example, the outer bag can be formed from a multilayer polymeric film comprising two layers of ethylene vinyl acetate with a layer of polyvinyl dichloride sandwiched therebetween.

The material from which the outer bag is formed typically is substantially impermeable to flatus gases and in particular the noxious components of flatus gases. Preferably therefore, in order to prevent the build up of flatus gases inside the ostomy bag assembly, the outer bag is provided with a flatus gas vent opening covered by a filter, which permits gases to exit the bag but filters out malodorous and noxious gases. Such filters are well known and need not be described here.

The inner bag is secured to the radially inner attachment zone, for example by welding or by adhesive bonding. Where adhesive bonding is used, the type of adhesive is selected so as to form a bond that cannot readily be broken by manual means. For example, the adhesive can be selected so that the strength of the adhesive bond is equal to or greater than the tear strength of the polymeric film from which the inner bag is made.

The inner bag may comprise one or more panels defining a bag structure having an opening for receiving stomal waste, and an annular mounting ring attached to the bag structure and surrounding the said opening, the annular mounting ring being bonded to the radially inner attachment zone of the adhesive flange. The annular mounting ring can be adhesively bonded by means of a non-peelable adhesive to the bag structure and by means of a non-peelable adhesive to the radially inner attachment zone. The term "non-peelable" as used herein means that the elements that are adhesively bonded together cannot readily be separated by manually peeling them apart. The non-peelable adhesives may be the same or different, the nature of the adhesive being selected according to the materials that it is intended to join. Examples of non-peelable adhesives include cyanoacrylate adhesives and rubber based adhesives, e.g. pressure-sensitive rubber based adhesives.

The term "rubber based adhesives" as used herein refers to both natural and synthetic rubbers. Examples of rubbers that can be included in rubber based adhesives include styrene block copolymer rubbers such as poly(styrenebutadiene-styrene) (SBS), poly(styrene-isoprene-styrene) (SIS), poly(styrene-ethylene-butylene-styrene) (SEBS) and other rubbers containing styrene and/or butadiene and/or ethylene and/or isoprene monomers. Such adhesives are widely available commercially, but see also WO2007/001743 for further information regarding rubber based adhesives.

In one embodiment, the annular mounting ring is attached to the bag structure of the inner bag by means of a cyanoacrylate adhesive and to the radially inner attachment zone of the adhesive flange by means of a rubber-based adhesive.

The annular mounting ring is typically formed from a polymeric material such as polyurethane, polyvinyl chloride, polyvinyl dichloride or a polyamide. More particularly, the annular mounting ring is formed from polyvinyl chloride.

The inner bag may be formed from a non-disposable waterproof material of a type described above for the outer bag, but preferably the inner bag is formed from a material that is biodegradable or disposable, such as polyvinyl alcohol. For example, the inner bag can be formed from a polymer, such as polyvinyl alcohol, of a type or grade that is slowly soluble in cold water but is more soluble in hot water. Examples of types of polyvinyl alcohol suitable for use in the fabrication of inner bags or liners are described in our earlier application WO94/12128.

In one embodiment, the inner bag comprises an inner layer formed from a hot water soluble grade of polyvinyl alcohol and an outer layer formed from a non-woven tissue comprising cold water-soluble polyvinyl alcohol fibres and water-insoluble polymer fibres (e.g. cellulosic or modified cellulosic fibres such as rayon fibres). The inner and outer layers are preferably secured together at their peripheries.

The radially inner and radially outer attachment zones are separated by means of an annular channel which extends through layers (c), (d) and (e) but not the first polymeric support layer (b). The term "annular" as used herein refers to a channel that surrounds the radially inner attachment zone and which may be circular but may alternatively be elliptical or polygonal or of an irregular shape. In one embodiment, the annular channel is circular or elliptical.

The channel is typically formed by means of die cutting through the layers (c), (d) and (e). The channel may, for example, be formed as a "kiss cut". The term "kiss cut" as used herein is used in its conventional sense to mean a cut through a laminar structure that extends through some but not all of the layers of the structure. A kiss cut can be formed using a die arranged to cut through a laminar structure to a pre-defined depth. Die cutting machines that can be programmed to form kiss cuts are widely available commercially.

In order to facilitate removal of the outer and inner bags from the adhesive flange, one or more tabs may be provided on the flange. For example, a primary tab comprising layers (c), (d) and (e) may be formed as a lateral extension to enable removal of the outer bag from the adhesive flange. A secondary tab, which facilitates removal of the inner bag from the adhesive flange can be formed as a lateral extension of an annular mounting ring of the inner bag. Alternatively, a line of weakness (for example a row of skip cuts) can be formed in the flange to provide an enclosed region (e.g. a thumb-shaped region) that can be torn away from the flange along with the inner bag.

The ostomy bag assemblies of the invention can be manufactured in a series of steps in which the adhesive flange is formed and the outer bags are then attached to and/or formed on the flange.

Accordingly, in another aspect, the invention provides a method for manufacturing an ostomy bag assembly of the invention as defined herein, which method comprises the steps of:
(i) providing a wafer comprising the first polymeric support layer (b), the bioadhesive layer (a) and a removable protective layer (f) for the bioadhesive;
(ii) die cutting a laminate blank comprising the heat sealable layer (c), the second polymeric support layer (d) and the weldable polymeric layer (e) from a web of coextruded polymeric layers;
(iii) placing the laminate blank on to the wafer;
(iv) heat sealing the laminate blank to the wafer to form an adhesive flange blank;
(v) die cutting the adhesive flange blank through layers (c), (d) and (e) but not the polymeric support layer (b) to give radially inner and outer attachment zones separated by the die cut;
(vi) bringing into contact with the die cut adhesive flange formed by step (v) a web of a material from which a panel of the outer bag is to be formed and welding the said web to the weldable polymeric layer of the radially outer attachment zone;
(vii) placing the inner bag on the adhesive flange, and bonding the inner bag to the radially inner attachment zone of the adhesive flange;
(viii) bringing into contact with the said web a further web of a material from which another panel of the outer bag is to be formed and outline welding the webs together so that they form the outer bag and enclose the inner bag; and thereafter
(ix) cutting the webs to release the ostomy bag assembly.

In process step (i) a datum hole may be punched in the wafer. The datum hole assists in the correct alignment of the components of the flange during the manufacturing process. Thus, in (iii), the laminate blank can be placed on the wafer so that the blank is disposed concentrically with respect to the datum hole.

The inner bag can be bonded to the radially inner attachment zone of the adhesive flange by welding or heat sealing to the weldable polymeric layer (e) or it can be adhesively bonded to the weldable polymeric layer (e). In a particular embodiment, the inner bag is adhesively bonded to the weldable layer (e).

To facilitate bonding to the weldable layer (e), the inner bag can be provided with an annular mounting ring as hereinbefore defined. The annular mounting ring is provided with a layer of adhesive for bonding to the weldable layer (e) and, during step (vii) of the process, the annular mounting ring is pressed against the weldable layer (e) to form an adhesive bond. The annular mounting ring can have a layer of adhesive applied thereto during the process step (vii) but, more usually, the annular mounting ring is pre-coated with a layer of adhesive. Where the annular mounting ring is pre-coated with a layer of adhesive, the adhesive may (although it need not be) be covered by a removable protective layer prior to taking part in the process, in which case the process may include an additional step of removing the removable protective layer.

The invention also provides adhesive flanges for use in manufacturing the ostomy bag assemblies of the invention.

Accordingly, in a further aspect, the invention provides an adhesive flange for use in manufacturing an ostomy bag assembly of the invention as defined herein, wherein the adhesive flange is of laminar construction and comprises, in sequence, from a body-contacting surface outwards:
(a) a bioadhesive layer for securing the flange to a body surface of a patient about the stomal opening;
(b) a first polymeric support layer attached to the bioadhesive layer (a);
(c) a heat sealable layer having a heat sealable adhesive surface which releasably bonds the heat sealable layer to the first polymeric support surface;
(d) a second polymeric support layer bonded to the heat sealable layer;
(e) a weldable polymeric layer to which inner and outer bags can be attached, the weldable polymeric layer being bonded to the second polymeric support layer;
the adhesive flange having an annular channel which surrounds the means defining the orifice and extends through layers (c), (d) and (e) so as to form radially inner and outer attachment zones separated by the annular channel, the radially inner attachment zone being attachable to an inner bag and the radially outer attachment zone being attachable to an outer bag.

The invention also provides a method of manufacturing the adhesive flanges of the invention as defined herein, which method comprises the steps of:
(i) providing a wafer comprising the first polymeric support layer (b), the bioadhesive layer (a) and a removable protective layer (f) for the bioadhesive;
(ii) die cutting a laminate blank comprising the heat sealable layer (c), the second polymeric support layer (d) and the weldable polymeric layer (e) from a web of coextruded polymeric layers;
(iii) placing the laminate blank on to the wafer;
(iv) heat sealing the laminate blank to the wafer to form an adhesive flange blank; and
(v) die cutting the adhesive flange blank through layers (c), (d) and (e) but not the polymeric support layer (b) to give radially inner and outer attachment zones separated by the die cut.

Further aspects and embodiments of the invention will be apparent from the specific description below and the drawings.

### Brief Description of the Drawings

Figure 1 is a schematic sectional view of an ostomy bag assembly according to one embodiment of the invention.
Figure 2 is an enlarged view of the region A in Figure 1 showing the layer structure of the ostomy bag assembly in the region of the attachment of the inner bag.
Figure 3 is a schematic sectional view, not to scale, showing the layer structure of the ostomy bag assembly of Figures 1 and 2.
Figure 4 is a plan view of an adhesive flange forming part of the ostomy bag assembly of Figures 1, 2 and 3.

### Detailed Description of the Invention

The invention will now be described in more detail, but not limited, by reference to the specific embodiment illustrated in the drawings.

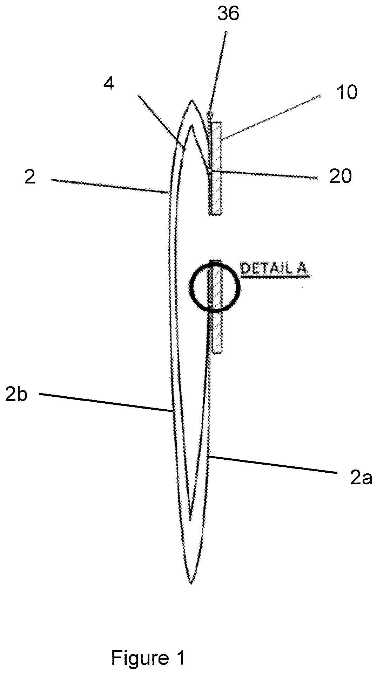

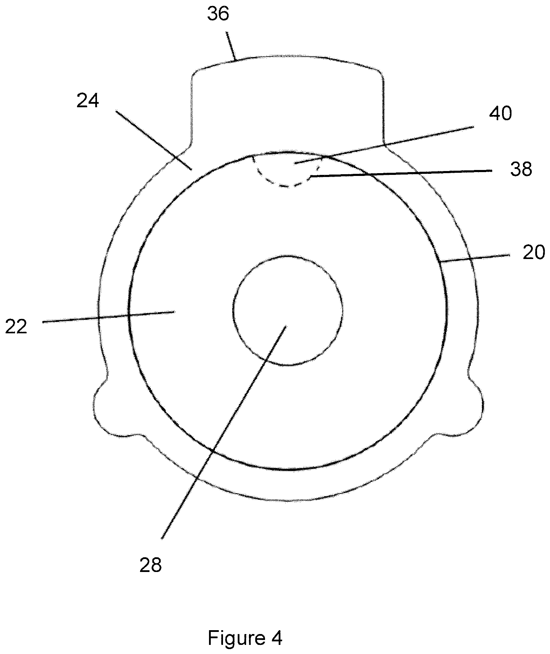

Referring now to the drawings, Figures 1 to 4 show an ostomy bag assembly according to a first embodiment of the invention.

The ostomy bag assembly of Figures 1 to 4 comprises an outer bag 2 and an inner bag 4 attached to an adhesive flange 6.

The adhesive flange 6 comprises a polymeric backing film (first polymeric support layer) 8 which, in this embodiment is formed from polyurethane and has a thickness of approximately 30 µm. Supported on the backing film 8 is a layer 10, approximately 0.6 mm to 0.9 mm thick, of a hydrocolloid adhesive. The hydrocolloid adhesive, which may be of conventional type, serves to secure the ostomy bag to the body of a patient. A siliconised paper release layer 12 covers the hydrocolloid adhesive layer and protects the adhesive layer against damage and/or drying out prior to use of the bag.

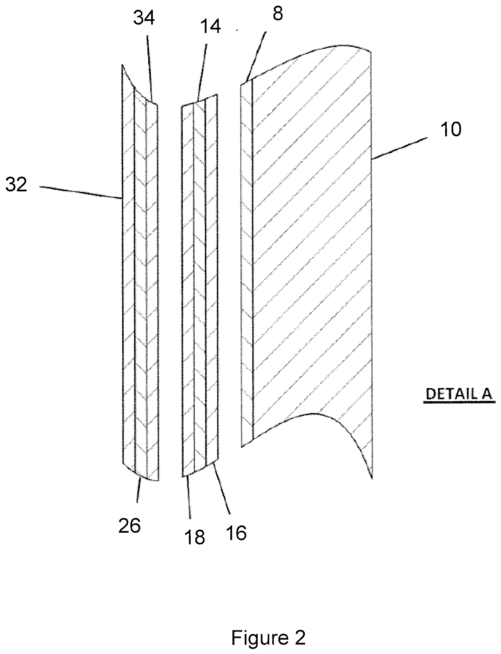

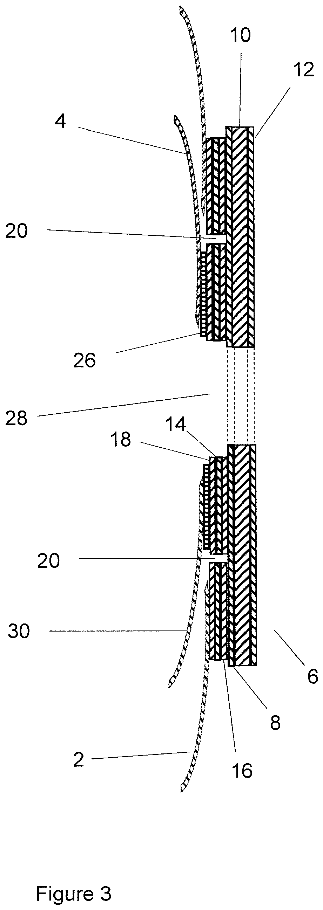

The layer structure of the adhesive flange can be seen in more detail in Figures 2 and 3. Thus, attached to the polyurethane backing film is co-extruded multilayer polymeric material which, in the particular embodiment illustrated, consists of a central layer 14 of Surlyn® sandwiched between two layers 16 and 18 of ethylene vinyl acetate (EVA). One of the EVA layers (18) is present in the form of a film and the other (16) is present as a layer of an EVA copolymer adhesive emulsion. The EVA adhesive-coated co-extruded multilayer polymeric material can be, for example, PerfecSeal® coated PerfecFlex ® medical forming film available from Perfecseal Limited of Londonderry, UK.

With reference to the claims and statements of invention herein, the siliconised release paper 12, hydrocolloid layer 10, polyurethane backing film 8, EVA adhesive emulsion 16, Surlyn ® layer 14 and EVA layer 18 correspond to layers (f), (a), (b), (c), (d) and (e) respectively.

The EVA adhesive emulsion layer 16 is bonded to the polyurethane backing film 8 by applying heat with an annular heat sealing tool at a temperature of 120 °C to 160 °C for a period of about 2 to 5 seconds. The EVA adhesive functions as a hot melt adhesive that forms a bond which, whilst easily strong enough to withstand any forces to which it is subjected during use, can subsequently be peeled apart using reasonable manual force.

During manufacture of the adhesive flange, a die cutting tool is used to create an annular kiss cut 20 which extends through the EVA layer 18, the Surlyn® layer 14 and the EVA adhesive emulsion layer 16 but not through the polyurethane backing film 8. The annular kiss cut, which extends in a circle around the flange, serves to divide the bag-side surface of the adhesive flange into concentric regions defining a radially inner attachment zone 22 and a radially outer attachment zone 24.

Extending outwardly from the outer attachment zone is a tab 36 which acts as primary release tab to facilitate the removal of the outer bag after use. A skip cut 38, which also extends through the EVA layer 18, the Surlyn® layer 14 and the EVA adhesive emulsion layer 16 but not through the polyurethane backing film 8, defines a secondary release tab 40 that can be used to assist removal of the inner bag after use.

The outer bag 2 is firmly bonded to the EVA layer 18 in the radially outer attachment zone 24 by welding, for example by Rf welding. This ensures a secure bond between annular bonding element and outer bag which cannot be disrupted without tearing the fabric of the outer bag.

The inner bag 4 is provided with a PVC mounting ring 26 which surrounds the opening 28 in the inner bag. The PVC ring is firmly bonded to the panel 30 of the inner bag by means of a layer 32 of cyanoacrylate adhesive. The strength of the bond is such that the panel of bag material cannot readily be removed from the PVC ring without damaging the structure of the bag. The PVC ring is bonded to the EVA layer 18 in the radially inner attachment zone by means of a layer 34 of rubber based adhesive. Adhesive layers 32 and 34 are not shown separately in Figure 3 but form part of the element identified in Figure 3 by the numeral 26.

The outer bag 2 in this embodiment can be formed from materials well known for the construction of ostomy bags. Thus, for example, it can be formed from a tough, flexible, transparent, waterproof material such as polyvinyl dichloride (PVDC), ethylene vinyl acetate (EVA), related materials and combinations thereof in known fashion, one particular material being the EVA/PVDC/EVA film available from Sealed Air of Saddle Brook, New Jersey, US under the trade name Cryovac MF514.

In the embodiment shown, the outer bag is formed from a pair of panels 2a and 2b formed from the flexible waterproof material, one panel 2a being cut so as to form an opening which is aligned with the opening 28 in the inner bag. The inner edge of the panel 2a, i.e. the region surrounding the opening, is welded to the radially outer attachment zone 24. The other panel 2b has the same outer periphery as panel 2a, but no opening. The two panels 2a and 2b are secured together around their respective peripheries by welding, (for example Rf welding) or by means of adhesive. Attached to the panels 2a and 2b by welding around their respective peripheries are panels (not shown) of a fibrous non-woven material such as a non-woven polyethylene fabric which serve as a comfort layer, providing a warmer and less harsh feeling against the skin of the patient.

The polymeric materials from which the panels 2a and 2b are formed act as a barrier to gases, and in particular flatus gases. Therefore, in order to prevent ballooning of the ostomy bag through the build up of flatus gases inside the bag, the outer bag is usually provided with a small opening (not shown) covered by a flatus filter (also not shown) which is welded to both the panel 2b and the comfort layer.

The inner bag 4 can be formed from two pairs of panels of polymeric material, welded together along their peripheries wherein the inner pair of panels are formed from a mechanically tough warm water soluble grade of polyvinyl alcohol film, for example LA40 film available from Aichello, Japan, and the outer pair of panels are formed from a fibrous non-woven tissue formed from cold water soluble polyvinyl alcohol fibres and rayon fibres, which disintegrates in water. However, in the drawings, for simplicity, only a single pair of panels is shown.

In use, faecal material from a stomal opening passes through the opening in the flange and the opening 28 in the inner bag into the interior of the inner bag 4. When the inner bag 4 is full, the outer bag 2 together with its attached portions of the EVA layer 18, the Surlyn® layer 14 and the EVA adhesive emulsion layer 16, can be peeled away from the polyurethane backing film 8. The remaining part of the flange and the inner bag may then be disposed of by flushing down a WC and the outer bag disposed of through normal domestic waste channels. A new assembly of inner and outer bag and adhesive flange may then be applied to the patient.

Because the inner bag is formed from materials that are soluble or disintegratable in water, and the hydrocolloid adhesive of the flange is also soluble or erodible in water, the sub-assembly of flange and inner bag disintegrates during flushing and subsequent passage through waste pipes leaving as a residue only the thin polyurethane backing film 8 and any insoluble fibres in the material from which the inner bag is formed.

However, as an alternative, the inner bag and its attached portions of the EVA layer 18, the Surlyn® layer 14 and the EVA adhesive emulsion layer 16, can also be peeled away from the polyurethane backing film 8 so that the inner bag 4 and the remainder of the flange can be disposed of separately. This method of disposal may be preferred where there are more stringent restrictions on the materials that can be flushed down a WC, for example because the construction of the waste pipe is such that it is more liable to become blocked, or where the disposal of non-water dispersible materials is forbidden or impractical.

Thus, one advantage of the ostomy bag assembly of the invention is that it provides the user with greater flexibility in the manner in which the assembly is disposed of after use.

Another advantage of the ostomy bag assembly of the invention is that it can be manufactured by a largely automated production process requiring relatively little manual intervention.

A typical manufacturing process for the ostomy bag assemblies is described below.

Wafers or blanks which will become layers (f), (a) and (b) of the adhesive flange 6 are die cut from sheets of a trilaminar material consisting of the polyurethane backing film 8, hydrocolloid adhesive 10 and siliconised paper 12. The wafers can be prepared off site or manufactured *in situ.* The wafers are loaded into a magazine and are transferred on a rotating carousel to a cutting station where a datum hole is die cut in the centre of the wafer. The hole serves as the datum point for the alignment of the various components of the ostomy bag assembly later in the manufacturing process.

In a separate operation, a web of a coextruded multilayer film consisting of Surlyn® sandwiched between two layers of ethylene vinylacetate (EVA), one of which is in the form of an EVA copolymer adhesive emulsion, is die cut to form discs of material that will become layers (c), (d) and (e) of the adhesive flange.

Each disc is then automatically conveyed to another work station where it is placed over an adhesive flange wafer so that the disc is concentric with the datum hole in the wafer. Heat and pressure are then applied to the disc to form a heat seal between the EVA copolymer adhesive emulsion layer of the disc and the polyurethane backing film 8 of the wafer. At the same time, or shortly afterwards, an annular channel or kiss cut is cut into the disc by means of a die cutter so that the kiss cut extends through the EVA and Surlyn layers 18, 14 and 16 but not through the polyurethane backing film 8.

Once the heat seal has been created, the sub-assembly of disc and adhesive flange wafer, which together form an adhesive flange blank constituting layers (f), (a), (b), (c), (d) and (e) of the adhesive flange, is removed, turned over and placed on a tray to cool with the layer (e) facing down so as to prevent curling.

After cooling, the die cut adhesive flange blanks are loaded into a magazine with the EVA layer 18 facing up and transferred to a separate machine for creating the ostomy bags.

In a first step in the creation of the ostomy bags, a first web of a non-woven fabric (from which a comfort panel (not shown) is made) is die cut to form a series of circular holes. A second web, which is formed from an EVA/PVDC/EVA film (which will become panel 2a) is then die cut with a series of holes of a smaller diameter than the holes in the first web. The first and second webs are then secured together by means of peripheral tack welds.

The adhesive flanges are then transferred from their magazine to a welding station where they are successively welded to the second web so that each sub-assembly surrounds one of the holes in the web.

The first and second webs carrying the adhesive flange blanks pass through a further processing station where pre-formed inner bags are adhesively bonded to the flange blanks. Each inner bag has a strengthening ring of PVC surrounding its opening, the PVC mounting ring being secured to the inner bag panel by means of a cyanoacrylate adhesive. The PVC ring is pre-coated with a rubber adhesive and is bonded to the flange blank with the application of pressure and optionally heat. In the present example, PVC rings coated with a rubber based adhesive (product code F277) were obtained from Avery Dennison and were then used in the manufacture of the inner bags.

At a separate filter welding station, a third web of material, from which the panel 2b will be formed, and a fourth web of material, from which a comfort panel (not shown) will be formed, are brought together and a filter is welded to the surface of the third web. The welding operation is carried out for a period of time sufficient to ensure that the fourth web is also welded to the third web in the region of the filter. The region over the filter where the third and fourth webs are welded together is then perforated to form an exit hole for flatus gases passing through the filter.

Once the filter has been affixed, the first, second, third and fourth webs are passed through another welding station where the four webs are outline welded together (the outline of the weld defining the shape of the ostomy bag). The webs are then cut around the outer edge of the outline to release the completed ostomy bag assembly from the webs. The completed ostomy bag assemblies may then be inspected and packed.

During the assembly of the ostomy bag, a further and optional cutting step may be employed in which the datum hole is enlarged to a size suitable for fitting about a stomal opening. During this step, differently sized cutters may be used for different batches thereby enabling the creation of a range of ostomy bags with different sizes of opening.

### Equivalents

It will readily be apparent that numerous modifications and alterations may be made to the specific embodiments of the invention described above without departing from the scope of the invention. All such modifications and alterations are intended to be embraced by this application.

## Claims

1. An ostomy bag assembly comprising outer (2) and inner bags (4) secured to one side of an adhesive flange (6); the adhesive flange (6) having means defining an orifice (28) to enable bodily waste from a stomal opening to be received by the inner bag (4); wherein the adhesive flange is of laminar construction and comprises, in sequence, from a body-contacting surface outwards:
(a) a bioadhesive layer (10) for securing the flange to a body surface of a patient about the stomal opening;
(b) a first polymeric support layer (8) attached to the bioadhesive layer (a);
(c) a heat sealable layer (16) having a heat sealable adhesive surface which releasably bonds the heat sealable layer to the first polymeric support surface; and
(d) a second polymeric support layer (14) bonded to the heat sealable layer (16);
(e) a weldable polymeric layer (18) to which the inner (4) and outer bags (2) are attached, the weldable polymeric layer (18) being bonded to the second polymeric support layer (14);
the adhesive flange (6) having an annular channel (20) which surrounds the means defining the orifice and extends through layers (c), (d) and (e) so as to form radially inner (22) and outer attachment zones (24) separated by the annular channel (20), the inner bag (4) being attached to the radially inner (22) attachment zone and the outer bag (2) being attached to the radially outer attachment zone (24);
and wherein the inner (4) and outer bags (2), together with attached portions of layers (c), (d) and (e) can each be peeled away from the first polymeric support layer (b).

2. An ostomy bag assembly according to claim 1 wherein the first polymeric support layer (b) (8) comprises a layer of polyurethane or polyamide film.

3. An ostomy bag assembly according to claim 1 or claim 2 wherein the heat sealable layer (c) (16) has a heat sealable adhesive surface comprising an ethylene vinyl acetate copolymer adhesive.

4. An ostomy bag assembly according to any one of claims 1 to 3 wherein the second polymeric support layer (d) (14) is formed from a polyamide.

5. An ostomy bag assembly according to any one of claims 1 to 4 wherein the weldable polymeric layer (e) (18) is formed from an ethylene polymer or copolymer.

6. An ostomy bag assembly according to claim 5 wherein the weldable polymeric layer (e) (18) is formed from ethylene vinyl acetate copolymer.

7. An ostomy bag assembly according to any one of claims 1 to 6 wherein the annular channel (20) has been formed by means of die cutting through the layers (c), (d) and (e).

8. An ostomy bag assembly according to any one of claims 1 to 7 wherein the annular channel (20) is circular or elliptical in plan.

9. A method for manufacturing an ostomy bag assembly as defined in any one of claims 1 to 8, which method comprises the steps of:
(i) providing a wafer comprising the first polymeric support layer (b) (8), the bioadhesive layer (a) (10) and a removable protective layer (f) (12) for the bioadhesive;
(ii) die cutting a laminate blank comprising the heat sealable layer (c) (16), the second polymeric support layer (d) (14) and the weldable polymeric layer (e) (18) from a web of coextruded polymeric layers;
(iii) placing the laminate blank on to the wafer;
(iv) heat sealing the laminate blank to the wafer to form an adhesive flange blank;
(v) die cutting the adhesive flange blank through layers (c), (d) and (e) but not the polymeric support layer (b) to give radially inner (22) and outer attachment zones (24) separated by the die cut;
(vi) bringing into contact with the die cut adhesive flange formed by step (v) a web of a material from which a panel of the outer bag (2) is to be formed and welding the said web to the weldable polymeric layer of the radially outer attachment zone (24);
(vii) placing the inner bag (4) on the adhesive flange (6), and bonding the inner bag (4) to the radially inner attachment zone (22) of the adhesive flange;
(viii) bringing into contact with the said web a further web of a material from which another panel of the outer bag (2) is to be formed and outline welding the webs together so that they form the outer bag and enclose the inner bag; and thereafter
(ix) cutting the webs to release the ostomy bag assembly.

10. A method according to claim 9 wherein, in step (vii), the inner bag is adhesively bonded to the adhesive flange (6).

11. An adhesive flange (6) for use in manufacturing an ostomy bag assembly as defined in any one of claims 1 to 8, wherein the adhesive flange (6) is of laminar construction and comprises, in sequence, from a body-contacting surface outwards:
(a) a bioadhesive layer (10) for securing the flange to a body surface of a patient about the stomal opening;
(b) a first polymeric support layer (8) attached to the bioadhesive layer (a);
(c) a heat sealable layer (16) having a heat sealable adhesive surface which releasably bonds the heat sealable layer to the first polymeric support surface;
(d) a second polymeric support layer (14) bonded to the heat sealable layer (16);
(e) a weldable polymeric layer (18) to which inner (4) and outer bags (2) can be attached, the weldable polymeric layer (18) being bonded to the second polymeric support layer (14);
the adhesive flange (6) having an annular channel (20) which surrounds the means defining the orifice and extends through layers (c), (d) and (e) so as to form radially inner (22) and outer attachment zones (24) separated by the annular channel (20), the radially inner (22) attachment zone being attachable to an inner bag (4) and the radially outer attachment zone (24) being attachable to an outer bag (2).

12. A method of manufacturing an adhesive flange (6) as defined in claim 11, which method comprises the steps of:
(i) providing a wafer comprising the first polymeric support layer (b) (8), the bioadhesive layer (a) (10) and a removable protective layer (f) (12) for the bioadhesive;
(ii) die cutting a laminate blank comprising the heat sealable layer (c) (16), the second polymeric support layer (d) (14) and the weldable polymeric layer (e) (18) from a web of coextruded polymeric layers;
(iii) placing the laminate blank on to the wafer;
(iv) heat sealing the laminate blank to the wafer to form an adhesive flange blank; and
(v) die cutting the adhesive flange blank through layers (c), (d) and (e) but not the polymeric support layer (b) to give radially inner (22) and outer attachment zones (24) separated by the die cut.

## Patentansprüche

1. Stomabeutelanordnung umfassend einen äußeren (2) und inneren Beutel (4), die an einer Seite eines Klebeflansches (6) gesichert sind; wobei der Klebeflansch (6) Mittel aufweist, die eine Öffnung (28) definieren, um zu ermöglichen, dass körperliche Ausscheidungen von einer Stomaöffnung von dem inneren Beutel (4) aufgenommen werden können; wobei der Klebeflansch einen laminaren Aufbau hat und der Reihe nach von einer Körperkontaktoberfläche nach außen hin Folgendes umfasst:
(a) eine Bioklebstoffschicht (10) zur Sicherung des Flansches an eine Körperoberfläche eines Patienten um die Stomaöffnung herum;
(b) eine erste Polymerträgerschicht (8), die an der Bioklebstoffschicht (a) befestigt ist;
(c) eine heißsiegelbare Schicht (16), die eine heißsiegelbare Klebeoberfläche aufweist, die die heißsiegelbare Schicht lösbar mit der ersten Polymerträgerschicht verbindet; und
(d) eine zweite Polymerträgerschicht (14), die mit der heißsiegelbaren Schicht (16) verbunden ist;
(e) eine schweißbare Polymerschicht (18), an der der innere (4) und äußere Beutel (2) befestigt sind, wobei die schweißbare Polymerschicht (18) mit der zweiten Polymerträgerschicht (14) verbunden ist;
wobei der Klebeflansch (6) einen ringförmigen Kanal (20) aufweist, der die Mittel, die die Öffnung definieren, umgibt und der sich durch Schichten (c), (d) und (e) erstreckt, um eine radial innere (22) und äußere Befestigungszone (24) zu bilden, die durch den ringförmigen Kanal (20) getrennt sind, wobei der innere Beutel (4) an der radial inneren Befestigungszone (22) befestigt ist und der äußere Beutel (2) an der radial äußeren Befestigungszone (24) befestigt ist,;
und wobei der innere (4) und äußere Beutel (2), zusammen mit den befestigten Abschnitten der Schichten (c), (d) und (e), jeweils von der ersten Polymerträgerschicht (b) abgezogen werden können.

2. Stomabeutelanordnung nach Anspruch 1, wobei die erste Polymerträgerschicht (b) (8) eine Schicht aus Polyurethan- oder Polyamidfilm umfasst.

3. Stomabeutelanordnung nach Anspruch 1 oder Anspruch 2, wobei die heißsiegelbare Schicht (c) (16) eine heißsiegelbare Klebeoberfläche aufweist, die einen Ethylen-Vinylacetat-Copolymer-Klebstoff umfasst.

4. Stomabeutelanordnung nach einem der Ansprüche 1 bis 3, wobei die zweite Polymerträgerschicht (d) (14) aus einem Polyamid gebildet ist.

5. Stomabeutelanordnung nach einem der Ansprüche 1 bis 4, wobei die schweißbare Polymerschicht (e) (18) aus einem Ethylen-Polymer oder -Copolymer gebildet ist.

6. Stomabeutelanordnung nach Anspruch 5, wobei die schweißbare Polymerschicht (e) (18) aus einem Ethylen-Vinylacetat-Copolymer gebildet ist.

7. Stomabeutelanordnung nach einem der Ansprüche 1 bis 6, wobei der ringförmige Kanal (20) durch Stanzen durch die Schichten (c), (d) und (e) gebildet wurde.

8. Stomabeutelanordnung nach einem der Ansprüche 1 bis 7, wobei der ringförmige Kanal (20) in der Draufsicht kreisförmig oder elliptisch ist.

9. Verfahren zur Herstellung einer Stomabeutelanordnung nach einem der Ansprüche 1 bis 8, wobei das Verfahren die folgenden Schritte umfasst:
(i) Bereitstellen eines Wafers, der die erste Polymerträgerschicht (b) (8), die Bioklebstoffschicht (a) (10) und eine lösbare Schutzschicht (f) (12) für den Bioklebstoff umfasst;
(ii) Stanzen eines Laminatzuschnitts, der die heißsiegelbare Schicht (c) (16), die zweite Polymerträgerschicht (d) (14) und die schweißbare Polymerschicht (e) (18) umfasst, aus einer Bahn aus coextrudierten Polymerschichten;
(iii) Platzieren des Laminatzuschnitts auf den Wafer;
(iv) Heißsiegeln des Laminatzuschnitts auf den Wafer, um einen Klebeflanschzuschnitt zu bilden;
(v) Stanzen des Klebeflanschzuschnitts durch Schichten (c), (d) und (e), aber nicht durch die Polymerträgerschicht (b), um eine radial innere (22) und äußere Befestigungszone (24) zu ergeben, die durch das Stanzen getrennt sind;
(vi) Inkontaktbringen einer Bahn aus einem Material, aus dem ein Element des äußeren Beutels (2) gebildet werden soll, mit dem in Schritt (v) gebildeten ausgestanzten Klebeflansch und Schweißen der Bahn an die schweißbare Polymerschicht der radial äußeren Befestigungszone (24);
(vii) Platzieren des inneren Beutels (4) auf den Klebeflansch (6) und Verbinden des inneren Beutels (4) mit der radial inneren Befestigungszone (22) des Klebeflansches;
(viii) Inkontaktbringen einer weiteren Bahn aus einem Material, aus dem ein weiteres Element des äußeren Beutels (2) gebildet werden soll, mit der Bahn und Konturschweißen der Bahnen, so dass sie den äußeren Beutel bilden und den inneren Beutel umschließen; und dann
(ix) Schneiden der Bahnen, um die Stomabeutelanordnung zu lösen.

10. Verfahren nach Anspruch 9, wobei in Schritt (vii) der innere Beutel mit dem Klebeflansch (6) klebend verbunden wird.

11. Klebeflansch (6) zur Verwendung bei der Herstellung einer Stomabeutelanordnung nach einem der Ansprüche 1 bis 8, wobei der Klebeflansch (6) einen laminaren Aufbau aufweist und der Reihe nach von einer Körperkontaktoberfläche nach außen hin Folgendes umfasst:
(a) eine Bioklebstoffschicht (10) zur Befestigung des Flansches an einer Körperoberfläche eines Patienten um die Stomaöffnung herum;
(b) eine erste Polymerträgerschicht (8), die an der Bioklebstoffschicht (a) befestigt ist;
(c) eine heißsiegelbare Schicht (16), die eine heißsiegelbare Klebeoberfläche aufweist, die die heißsiegelbare Schicht mit der ersten Polymerträgeroberfläche lösbar verbindet;
(d) eine zweite Polymerträgerschicht (14), die mit der heißsiegelbaren Schicht (16) verbunden ist;
(e) eine schweißbare Polymerschicht (18), an der der innere (4) und äußere Beutel (2) befestigt werden können, wobei die schweißbare Polymerschicht (18) mit der zweiten Polymerträgerschicht (14) verbunden wird;
wobei der Klebeflansch (6) einen Ringkanal (20) aufweist, der die Mittel, die die Öffnung definieren, umgibt und der sich durch die Schichten (c), (d) und (e) erstreckt, um eine radial innere (22) und äußere Befestigungszone (24) zu bilden, die durch den ringförmigen Kanal (20) getrennt sind, wobei die radial innere (22) Befestigungszone an einen inneren Beutel (4) befestigt werden kann und die radial äußere Befestigungszone (24) an einen äußeren Beutel (2) befestigt werden kann.

12. Verfahren zur Herstellung eines Klebeflansches (6) nach Anspruch 11, wobei das Verfahren die folgenden Schritte umfasst:
(i) Bereitstellen eines Wafers, der die erste Polymerträgerschicht (b) (8), die Bioklebstoffschicht (a) (10) und eine lösbare Schutzschicht (f) (12) für den Bioklebstoff umfasst;
(ii) Stanzen eines Laminatzuschnitts, der die heißsiegelbare Schicht (c) (16), die zweite Polymerträgerschicht (d) (14) und die schweißbare Polymerschicht (e) (18) umfasst, aus einer Bahn aus coextrudierten Polymerschichten;
(iii) Platzieren des Laminatzuschnitts auf dem Wafer;
(iv) Heißsiegeln der Laminatzuschnitte an den Wafer, um einen Klebeflanschzuschnitt zu bilden; und
(v) Stanzen des Klebeflanschzuschnitts durch Schichten (c), (d) und (e), aber nicht durch die Polymerträgerschicht (b), um eine radial innere (22) und äußere Befestigungszone (24) zu ergeben, die durch das Stanzen getrennt sind.

## Revendications

1. Ensemble poche de stomie comprenant des poches interne (4) et externe (2) fixées à un côté d'un rebord adhésif (6) ; le rebord adhésif (6) ayant des moyens définissant un orifice (28) pour permettre la réception des déchets corporels provenant d'une ouverture stomale par la poche interne (4) ; ledit rebord adhésif étant d'une construction stratifiée et comprenant, en séquence, depuis une surface en contact avec le corps vers l'extérieur :
(a) une couche bioadhésive (10) pour fixer le rebord à une surface corporelle d'un patient autour de l'ouverture stomale ;
(b) une première couche de support polymère (8) fixée à la couche bioadhésive (a) ;
(c) une couche thermoscellable (16) comportant une surface adhésive thermoscellable qui lie de manière amovible la couche thermoscellable à la première surface de support polymère ; et
(d) une seconde couche de support polymère (14) liée à la couche thermoscellable (16) ;
(e) une couche polymère soudable (18) à laquelle sont fixées les poches interne (4) et externe (2), la couche polymère soudable (18) étant liée à la seconde couche de support polymère (14) ;
le rebord adhésif (6) comportant un canal annulaire (20) qui entoure les moyens définissant l'orifice et s'étend à travers les couches (c), (d) et (e) de manière à former des zones de fixation radialement interne (22) et externe (24) séparées par le canal annulaire (20), la poche interne (4) étant fixée à la zone de fixation radialement interne (22) et la poche externe (2) étant fixée à la zone de fixation radialement externe (24) ;
et lesdites poches interne (4) et externe (2), conjointement avec les portions fixées des couches (c), (d) et (e), pouvant chacune être décollées de la première couche de support polymère (b).

2. Ensemble poche de stomie selon la revendication 1, ladite première couche de support polymère (b) (8) comprenant une couche de film polyuréthane ou polyamide.

3. Ensemble poche de stomie selon la revendication 1 ou 2, ladite couche thermoscellable (c) (16) possédant une surface adhésive thermoscellable comprenant un adhésif de copolymère éthylène-acétate de vinyle.

4. Ensemble poche de stomie selon l'une quelconque des revendications 1 à 3, ladite seconde couche de support polymère (d) (14) étant formée à partir d'un polyamide.

5. Ensemble poche de stomie selon l'une quelconque des revendications 1 à 4, ladite couche polymère soudable (e) (18) étant formée à partir d'un polymère ou copolymère d'éthylène.

6. Ensemble poche de stomie selon la revendication 5, ladite couche polymère soudable (e) (18) étant formée à partir d'un copolymère éthylène-acétate de vinyle.

7. Ensemble poche de stomie selon l'une quelconque des revendications 1 à 6, ledit canal annulaire (20) ayant été formé au moyen d'une découpe à l'emporte-pièce à travers les couches (c), (d) et (e).

8. Ensemble poche de stomie selon l'une quelconque des revendications 1 à 7, ledit canal annulaire (20) étant circulaire ou elliptique dans le plan.

9. Procédé de fabrication d'un ensemble poche de stomie tel que défini dans l'une quelconque des revendications 1 à 8, lequel procédé comprenant les étapes de :
(i) fourniture d'une plaquette comprenant la première couche de support polymère (b) (8), la couche bioadhésive (a) (10) et une couche de protection amovible (f) (12) pour le bioadhésif ;
(ii) découpe à l'emporte-pièce d'une ébauche stratifiée comprenant la couche thermoscellable (c) (16), la seconde couche de support polymère (d) (14) et la couche polymère soudable (e) (18) à partir d'une bande de couches polymères coextrudées ;
(iii) disposition de l'ébauche stratifiée sur la plaquette ;
(iv) thermoscellage de l'ébauche stratifiée à la plaquette pour former une ébauche de rebord adhésif ;
(v) découpe à l'emporte-pièce de l'ébauche de rebord adhésif à travers les couches (c), (d) et (e) mais non la couche de support polymère (b) pour donner des zones de fixation radialement interne (22) et externe (24) séparées par la découpe à l'emporte-pièce ;
(vi) mise en contact avec le rebord adhésif découpé à l'emporte-pièce formé par l'étape (v) d'une bande d'un matériau à partir duquel un pan de la poche externe (2) doit être formé, et soudage de ladite bande à la couche polymère soudable de la zone de fixation radialement externe (24) ;
(vii) disposition de la poche interne (4) sur le rebord adhésif (6), et collage de la poche interne (4) à la zone de fixation radialement interne (22) du rebord adhésif ;
(viii) mise en contact avec ladite bande d'une bande supplémentaire d'un matériau à partir duquel un autre pan de la poche externe (2) doit être formé, et soudage de contours des bandes ensemble de façon à ce qu'elles forment la poche externe et entourent la poche interne ; et par la suite
(ix) découpe des bandes pour libérer l'ensemble poche de stomie.

10. Procédé selon la revendication 9, dans l'étape (vii), ladite poche interne étant liée de manière adhésive au rebord adhésif (6).

11. Rebord adhésif (6) destiné à la fabrication d'un ensemble poche de stomie tel que défini dans l'une quelconque des revendications 1 à 8, ledit rebord adhésif (6) étant de construction laminaire et comprenant, en séquence, depuis une surface en contact avec le corps vers l'extérieur :
(a) une couche bioadhésive (10) pour fixer le rebord à une surface corporelle d'un patient autour de l'ouverture stomale ;
(b) une première couche de support polymère (8) fixée à la couche bioadhésive (a) ;
(c) une couche thermoscellable (16) comportant une surface adhésive thermoscellable qui lie de manière amovible la couche thermoscellable à la première surface de support polymère ; et
(d) une seconde couche de support polymère (14) liée à la couche thermoscellable (16) ;
(e) une couche polymère soudable (18) à laquelle peuvent être fixées les poches interne (4) et externe (2), la couche polymère soudable (18) étant liée à la seconde couche de support polymère (14) ;
le rebord adhésif (6) comportant un canal annulaire (20) qui entoure les moyens définissant l'orifice et s'étend à travers les couches (c), (d) et (e) de manière à former des zones de fixation radialement interne (22) et externe (24) séparées par le canal annulaire (20), la zone de fixation radialement interne (22) pouvant être fixée à une poche interne (4) et la zone de fixation radialement externe (24) pouvant être fixée à une poche externe (2).

12. Procédé de fabrication d'un rebord adhésif (6) tel que défini dans la revendication 11, lequel procédé comprenant les étapes de :
(i) fourniture d'une plaquette comprenant la première couche de support polymère (b) (8), la couche bioadhésive (a) (10) et une couche de protection amovible (f) (12) pour le bioadhésif ;
(ii) découpe à l'emporte-pièce d'une ébauche stratifiée comprenant la couche thermoscellable (c) (16), la seconde couche de support polymère (d) (14) et la couche polymère soudable (e) (18) à partir d'une bande de couches polymères coextrudées ;
(iii) disposition de l'ébauche stratifiée sur la plaquette ;
(iv) thermoscellage de l'ébauche stratifiée à la plaquette pour former une ébauche de rebord adhésif ; et
(v) découpe à l'emporte-pièce de l'ébauche de rebord adhésif à travers les couches (c), (d) et (e) mais non la couche de support polymère (b) pour donner des zones de fixation radialement interne (22) et externe (24) séparées par la découpe à l'emporte-pièce.
